# EUROPEAN PATENT SPECIFICATION

(11) **EP 2 198 916 B1**
(45) Date of publication and mention of the grant of the patent: **16.09.2015**
(21) Application number: 09179318.2
(22) Date of filing: 15.12.2009
(51) Int. Cl.: A61N 1/365, A61B 5/00

(54) **Implantable telemetric device for heart monitoring**
Implantierbare telemetrische Vorrichtung zur Herzüberwachung
Dispositif télémétrique implantable pour la surveillance du coeur

(30) Priority: 16.12.2008 IT BO20080749
(43) Date of publication of application: 23.06.2010
(73) Proprietor: Tre Esse Progettazione Biomedica S.r.l, 40138 Bologna (IT)
(72) Inventor: PLICCHI, Gianni, I-40125, BOLOGNA (IT); MARCELLI, Emanuela, I-40125, BOLOGNA (IT); CERCENELLI, Laura, I-60030, CASTELBELLINO, Province of Ancona (IT)
(74) Representative: Porsia, Attilio

(56) References cited:
- EP-A2- 0 655 260
- WO-A1-99/35963
- WO-A2-2006/086435
- WO-A2-2006/127594
- US-A- 5 609 612
- US-B1- 7 139 609
- MARCELLI E ET AL: "Assessment of cardiac rotation by means of gyroscopic sensors" COMPUTERS IN CARDIOLOGY, 2008, IEEE, PISCATAWAY, NJ, USA, 14 September 2008 (2008-09-14), pages 389-392, XP031406573 ISBN: 978-1-4244-3706-1

## Description

The invention relates to a cardiac device which is associated with a catheter, which can be implanted endocardially or epicardially, and which is provided with means for detecting electromechanical parameters of the heart for diagnostic and/or therapeutic purposes, for example for monitoring the heart function in order to detect the onset of any alarm conditions which require the use of therapeutic protocols of the pharmacological type, or for controlling implantable therapeutic devices such as pacemakers, defibrillators or other devices.

Italian patent application BO2005A000165 filed on 17 March 2005 and granted on 03 July 2009 under No. 1 362 862 describes an implantable cardiac device which is essentially provided with a sensor for detecting the rotation of the heart and for generating an electrical signal representing this rotation, and is provided with means which process this signal and compare it with basal levels, and which are designed to interact with any implantable or external device for diagnostic and/or therapeutic and/or monitoring purposes. The sensor which detects the rotation of the heart is mounted on the distal end of a catheter which is implanted in the epicardium or in the endocardium or intramurally. The sensor may be composed of a small vibrating gyroscope which detects with high precision the Coriolis force due to the rotation rate of the heart and which produces a signal corresponding to this rotation rate.

US patent 7.445.605 describes a device for monitoring cardiac function in humans, provided with at least one motion sensor for detecting the movement of the heart, which, among other reported possibilities, can be composed of a rotation sensor and which comprises analytical means connected to said sensor for operation, these means receiving the signal generated by this component in order to process it and compare it with a basal level of the heart movement, this level being for example that of the normal movement of the healthy heart, and being introduced into the system as the known reference and threshold level.

There is no specific reference in the prior art to the use of specific signals which are correlated with the physiological function of the patient to be associated with the heart movement signal, except in relation to the intracardiac electrocardiogram, or, in an indirect way, in patent US 7.445.605, the mention of the possibility of combining the motion sensor with an implantable device such as a pacemaker and/or defibrillator, in which the parameters usually detected by said implantable devices are, in addition to the endocavitary electrocardiogram, respiratory parameters deduced from the measurement of endocavitary impedance. However, none of these signals mentioned in the prior art, nor the intracardiac electrocardiogram, nor the respiration parameters which may be derived from an impedance measurement can provide information on the actual mechanical phases of heart function, particularly the actual mechanical phases of the start and end of systole and diastole, which can be ascertained only by methods which enable the closing phases of the mitral and aortic valves to be detected, or by detecting the heart sounds, namely the first heart sound (FHS) and the second heart sound (SHS), these being detected by means of an implantable device. At present, these parameters can be derived from an echocardiogram or from external phonocardiography, in other words by methods not compatible with an autonomous implanted device, or can be derived from microaccelerometers positioned directly in the tip of an implantable electrode for cardiac stimulation, as described in US patent 5 609 612, or positioned in a subcutaneous casing similar to those used for implantable cardiac electrostimulators, as described in US patent 6 869 404B2, to which reference will mainly be made. In these prior patents, the said signals relating to the heart sounds are correlated and processed as a function of the electrical signals relating to the electrocardiogram, with which they must be suitably synchronized. The detection of the endocavitary electrogram, or the detection of the subcutaneous or surface ECG, would not be sufficient to identify precisely the start and end of the cardiac phases of systole and diastole, since the temporal correspondence between the electrical phases (detectable from the ECG) and the mechanical phases (systole and diastole) is not always identical and changes as a function of pathologies, the development of these, and contingent working conditions of the heart. For example, the time elapsing between the peak of the R wave and the start of mechanical systole varies greatly between a patient with a narrow QRS wave and a patient with a left bundle branch block. In the case of a left bundle branch block, there is a pathological delay between the QRS and the start of the systolic ejection phase, as well as an increase in the isovolumic contraction phase which precedes systole.

It is well known that in healthy subjects the cardiac rotation follows a specific pattern within the cardiac cycle in order to be effective: during isovolumic contraction all short-axis levels from base to apex rotate counterclockwise, when viewed from the apex, while, during ejection, the apex continues counterclockwise while the base reverses direction; in diastole, during isovolumic relaxation, the shearing forces built up during systole by cardiac torsion result in rapid untwisting which is thought to contribute to diastolic suction (see "J Biomech 1996; 29(6): 745-52", "Am J Physiol Heart Circ Physiol 2000;278(4): H1117-23", "Ann Biomed Eng 1986; 14(6): 547-62"). Alterations in the normal pattern or magnitude of the cardiac rotation have been associated with cardiovascular diseases, such as chronic heart failure (see "Eur J of Heart Fail 2004;6:715-722"), tachycardia-induced dilated cardiomyopathy (see "J Thorac Cardiovasc Surg 2002;124:43-9"), dilated cardiomyopathy (see "J Thorac Cardiovasc Surg 2003;126:48-55"), hypertrophic cardiomiopathy (see "Circulation 1992; 86(6):1919-28"), myocardial infarction (see "Coron Artery Dis 2000;11 (3):261-267") and aortic stenosis (see "Eur Heart J 2000; 21:582-589").
Thus, the analysis of the cardiac rotation signal with respect to systolic and diastolic phases is fundamental for the monitoring of cardiac function in patients with various degrees of heart failure (HF) or in non HF cardiopathic patients, who require, for example, monitoring of the effects of ischemia.
As for example, in cardiac insufficiency, there are often delay and/or dyssynchrony phenomena which are non-uniform mechanical movements of regions of the myocardium in the systolic and diastolic phases, which considerably reduce the overall mechanical efficiency of the heart, thus exacerbating and aggravating other cardiac insufficiency conditions. An important element of dyssynchrony has been identified in the time interval between the closing of the aortic valve (corresponding to the SHS) and the peak of the heart rotation signal at the end of systole (see "Am J Cardiol 2008;101:1163-1169"). If this dyssynchrony time interval can be determined, it will be possible to achieve suitable adaptation of both the pharmacological treatment and the instrumental treatment in the form of the implantation of a so-called "biventricular" cardiac electrostimulator, in which the electrostimulation parameters relating to the right and left ventricles have the function of resynchronizing the heart mechanics and can be optimized if there is a knowledge of this dyssynchrony interval, which must be minimized.

Prior art document WO 2006/086435 A2 discloses an implantable telemetric device for measuring electromechanical parameters of the heart, which comprises at least one acceleration sensor and corresponding processing means for detecting data relating to both vibrational and displacement motion of the heart and that comprises means which use these data for diagnostic and /or therapeutic and/or monitoring purposes. A similar tracking is provided in "Assessment of cardiac rotation by means of gyroscopic sensors", in Computer in Cardiology Conf. Proc., 35:389-392 IEEE, 2008.

Detection of mechanical vibrations which correspond to heart sounds are also disclosed in prior art document US 7 139 609 B1.

The invention is intended to overcome these and other drawbacks of the known art with an apparatus as described in the appended Claim 1 and subsequent dependent claims.

Further characteristics of the invention, and the advantages resulting therefrom, will be made clearer by the following description of a preferred embodiment of the invention, illustrated purely by way of non-limiting example in the figures on the four attached sheets of drawing, in which:
- Fig. 1 is a schematic illustration of the device according to the invention, also shown in relation to external control and programming equipment;

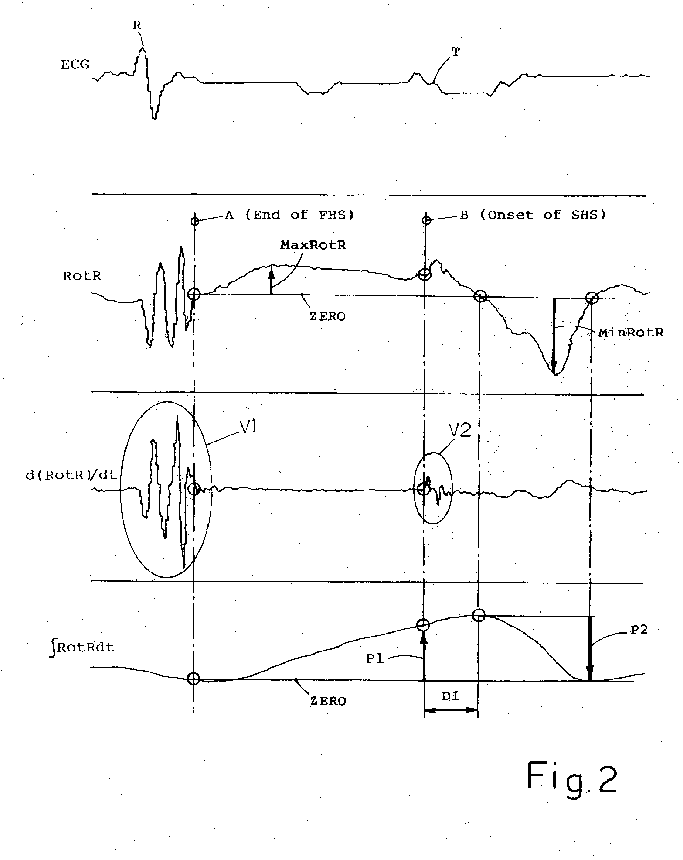

- Fig. 2 shows a plurality of analogue signals obtained during experiments conducted on experimental animals to demonstrate the way in which the broadband "Rotation Rate" (RotR) signal generated by the sensor used in the device according to the invention is processed by the device to produce, by an operation of derivation, the signal for the mechanical vibrations corresponding to the heart sounds [d(RotR)/dt] and to find, by an operation of integration, the heart rotation signal (jRotRdt) and an example, of combination of the said processed signals to identify correctly the peaks of rotation rate during the ejection phase (MaxRotR) and the relaxation/refilling phase (MinRotR) of the heart chamber, and any dyssynchrony intervals (DI) corresponding to the time interval between the peak of said rotation signal (∫RotRdt) and the second heart sound (SHS);

- Fig. 3 is a block diagram of the implantable device;
- Fig. 4 is a block diagram of the system for collecting and processing data using the device according to the invention.

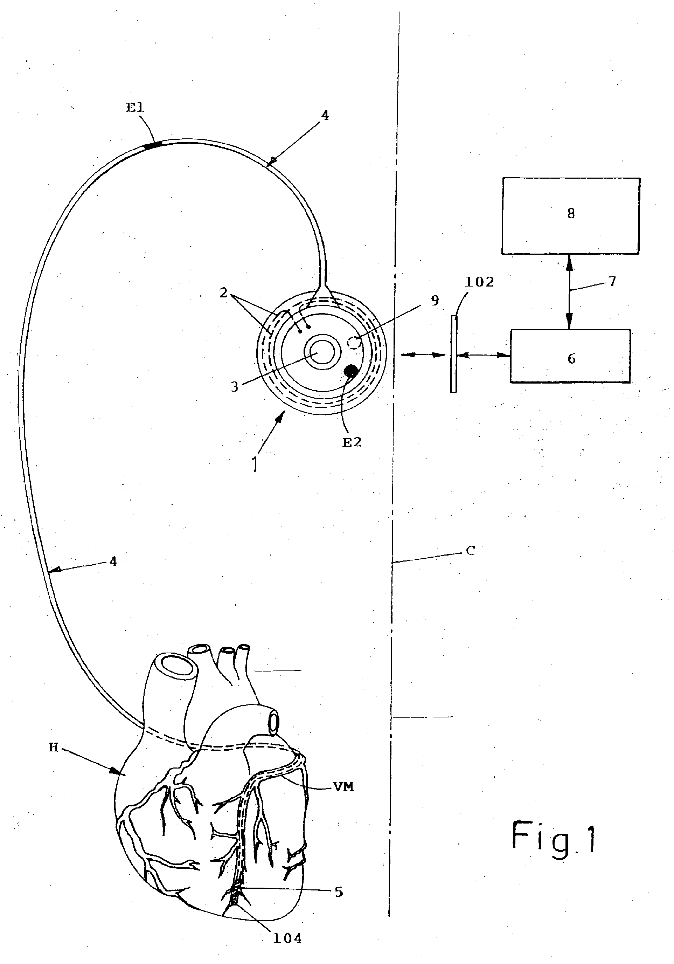

As shown in Figure 1, in a preferred embodiment the device according to the invention comprises a casing 1 which is to be placed under the skin C of the patient's body, and which is therefore made from a material with a biocompatible outer surface, such as polyurethane. The casing 1 is surrounded by the coil 2 of a system for receiving electrical energy by inductive coupling and for transmitting the detected data to the outside, while the casing 1 also houses an internal unit 3 containing the integrated circuits with the necessary hardware and software for data acquisition and transmission, and with a posture sensor 9, such as a three-dimensional accelerometer, sensitive to gravitational acceleration (see below).

The casing 1 also houses one of the electrodes E2 for detecting the electrocardiographic signal from the heart of the patient in whom the device in question is to be implanted.

A catheter 4 runs from the casing 1 and is implanted in the patient's cardiovascular system, with its distal end 104 placed as closely as possible to the apex of the heart H, next to the left ventricle, for example by insertion through the great cardiac vein VM. In an intermediate part of the catheter 4, for example, there is at least one other electrode E1 which, together with the aforesaid electrode E2, enables the electrocardiographic signal to be detected by the known ECG procedure (see below).

At the distal end 104 of the catheter 4 there is a sensor 5 for detecting the broadband signal of mechanical rotation rate, which is converted by the sensor to an electrical signal which, when processed by an operation of integration, supplies a signal relating to the rotation of the heart and, by an operation of derivation, supplies a signal relating to the mechanical vibrations corresponding to the heart sounds. Said sensor 5 can be composed, for example, of a miniature gyroscopic piezoelectric fork sensor, with a sensitivity range of approximately 0-1000 Hz, although it should be appreciated that other types of sensor can be used, provided that they are suitable for the purpose. For example, rotation sensors, rather than rotation rate sensors, can be used to obtain similar information simply by means of operations of derivation. In this case, the second derivative would correspond to the mechanical vibrations corresponding to the heart sounds as evaluated in the case described.

The implanted device can interact with an external programming and interrogation system, comprising a reception and transmission antenna 102 connected to a portable data collection unit 6, which can be provided with an alarm signalling device for responding to the detection of any anomalous data, and which, by means of any suitable data transfer means 7, can be connected by a wire or wireless link to an external server 8 provided with means for data acquisition, for the analysis of the data, and for commands and feedback signals to the patient.

Figure 2 shows the following intercorrelated signals detected during experiments on animals. The first signal, at the top of the figure, corresponds to an electrocardiogram or ECG, in which the R, S and T waves of a cardiac cycle are marked. The new sensor 5 used in the device according to the invention, which as stated has a sensitivity in the range from 0 to 1000 Hz, intrinsically generates a broadband signal which corresponds to the second signal RotR shown in Figure 2. By an operation of derivation, this signal RotR is processed to obtain the third signal d(RotR)/dt which contains the information relating to the mechanical vibrations corresponding on the heart sounds. The fourth signal, ∫RotRdt, corresponds to the angle of rotation of the heart and is obtained by an operation of integrating said signal RotR.

Figure 2 also shows:
The vibrations V1 and V2 corresponding to the FHS and SHS respectively;

The end line A corresponding to the end of the vibrations V1, i.e. the "End of FHS";

The end line B corresponding to the start of the vibrations V2, i.e. the "Onset of SHS".

Figure 2 also shows an example of combination of the said processed signals d(RotR)/dt and ∫RotRdt to characterize cardiac rotation with respect to systolic and diastolic phases, e.g. to identify:
MaxRotR (the peak of rotation rate during ejection);
MinRotR (the peak of rotation rate during relaxation/refilling);
The peak P1 of rotation during ejection;
The peak P2 of rotation during ventricular relaxation.

The automatic detection of the peak MaxRotR of the rotation rate of the cardiac apex, without reference to the mechanical phases of the heart cycle, is not reliable if measured as the absolute peak of the rotation rate signal, since this signal may show more than one peak. The peak of the rotation rate corresponding to the ejection phase is that which is found within a time interval between the first heart sound FHS and the second heart sound SHS, which can be detected from the d(RotR)/dt signal. The zero value of the rotation rate is found at the end of the said FHS, when the left ventricle is ready to start the blood ejection phase. This is because, at this instant, the heart has initiated the isovolumic contraction phase, making it possible to find the objective and reproducible zero point of each signal detected. In the absence of this important information, even the measurements of the positive and negative peaks would be affected by errors due to arbitrary assumptions relating to the zero reference point. This is the most well-defined signal and is essential for the precise and reproducible measurement of the peaks of the rotation rate and the peak of the rotation itself. As further example of combination of the said processed signals, by using the mechanical heart vibration signal d(RotR)/dt and the heart rotation signal ∫RotRdt it is possible to identify and signal correctly any dyssynchrony intervals (DI), corresponding to the time interval between the peak of said rotation signal and the closing of the aortic valve, identified by the mechanical vibrations corresponding to the SHS.

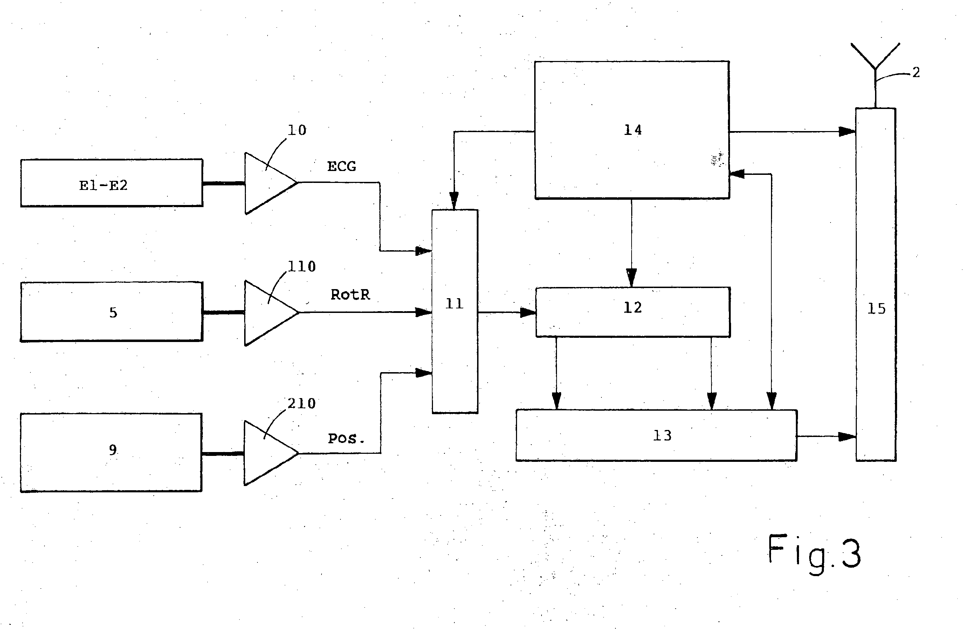

As shown in Figure 3, the electrodes E1 and E2 of Figure 1, used to acquire the patient's cardiac ECG, the sensor 5 for measuring the rotation and the mechanical vibrations corresponding to the heart sounds, and the three-dimensional sensor 9 for detecting the "Pos." data relating to the patient's posture are connected via corresponding amplifiers 10, 110, 210 to a multiplexer 11, which in turn is connected to an A/D converter 12, which samples the signals from the three different channels at an appropriate frequency, for example at a sampling frequency of about -1 kHz. The amplifier 110 transmits the broadband signal of the rotation rate of the heat to the multiplexer 11. The data from the multiplexer 11 are sent to an analogue/digital converter 12, and from there to a RAM 13 and to a control unit 14 which acquires and processes the data and which operates in a feedback circuit with the units 11, 12 and 13. Clearly, the scope of the invention also includes the variant embodiment in which the analogue signals from the three different channels E1-E2, 5 and 9 are digitized by suitable converters before being sent to a multiplexer 11, which in this case would be of a completely digital type. By means of the telemetric unit 15, the units 13 and 14 can interact with the outside via the antenna 2 of the device, as shown in Figure 1.

In the case of an implantable device which can also provide real-time treatment, the information detected by the system which has been described can be transmitted externally to other more complex processing systems, or can be processed internally and automatically for the purpose of therapeutic decision-making.

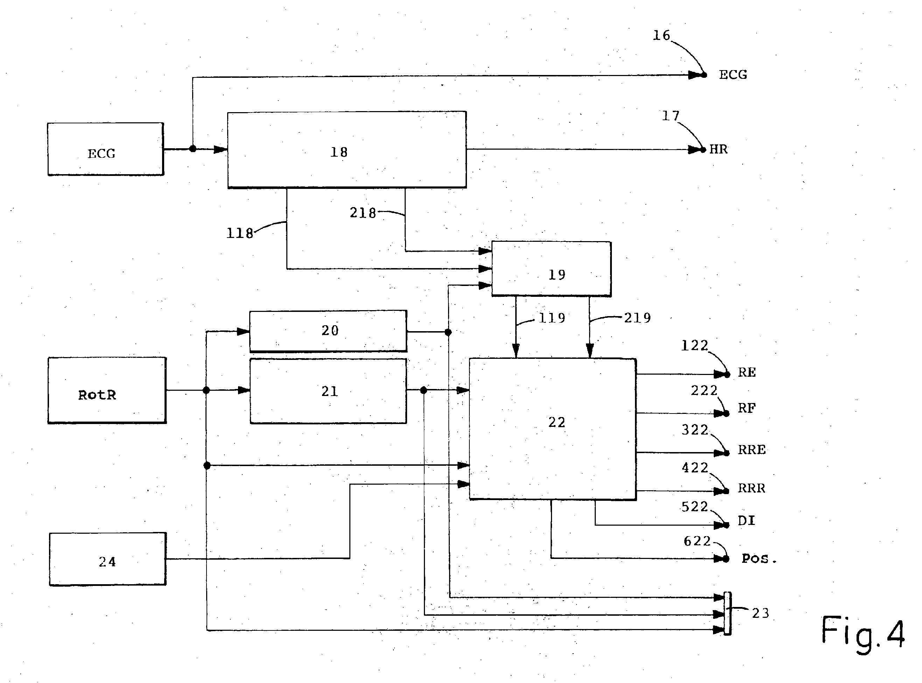

The block diagram in Figure 4 summarizes the system for processing the data detected, amplified and transmitted by the system of Fig. 3 described above. The ECG is made directly available via the output 16 for the morphological analysis and analysis of the heart rhythm. The unit 18 supplies the heart rate HR to the output 17. The unit 18 identifies the R and T waves from the ECG. The outputs 118 and 218 of the unit 18 transfer the R wave signal and the T wave signal, respectively, to the processing unit 19, to enable the unit 19 to detect the first heart sound FHS and the second heart sound SHS.

The broadband heart signal RotR is sent to the units 20 and 21, which supply the analogue signal d(RotR)/dt and the analogue rotation signal ∫RotRdt respectively. The first unit 20 is connected to the unit 19 in such a way that its outputs 119, 219 can identify the first heart sound FHS and the second heart sound SHS, which, together with the signal from the unit 21 and the signal RotR, are sent to the unit 22, which processes the various data for the evaluation of the parameters relating to the rotation of the heart, and which supplies the measurement of the rotation RE relating to the ejection phase of the heart chamber at the output 122, supplies the measurement of the rotation RF relating to the refilling of the heart chamber at the output 222, supplies the measurement of the peak rotation rate RRE relating to the ejection phase at the output 322, supplies the measurement of the peak rotation rate RRR relating to the phase of relaxation and refilling of the heart chamber at the output 422, supplies the measurement of DI, in other words any dyssynchrony interval as described above with reference to Figure 2, at the output 522, supplies the measurement of any other meaningful parameter derived from basic signal processing operations (i.e. sum, subtraction, multiplication, division, mean, calculation of the area underneath curves) and finally supplies the parameters relating to the patient's posture, obtained from the unit 24, at the output 622.

The analogue signals RotR, d(RotR)/dt and ∫RotRdt can be collected from the output 23.

There are evident advantages that can be obtained by the use of a single sensor 5 for supplying a signal relating to the rotation of the heart and to the heart sounds, which is not affected by external electrical signals, which can easily be processed without any need for synchronization with other physiological signals, and which is a source for the determination, by simple processing operations, of the aforesaid functions ∫RotRdt, d(RotR)/dt and for the estimation of any meaningful parameter (e.g. Dyssynchrony Interval DI) derived from the combination of said functions and from the application of simple signal processing operations to them. Clearly, the scope of the invention includes any solution other than that described which is capable of achieving the same ends, and therefore said sensor 5 can be replaced by or associated with one or more suitable motion sensors, such as accelerometers, capable of additionally detecting the translational movements of the heart in the radial and longitudinal directions, or other sensors of the gyroscopic type capable of detecting the rotation of the heart wall at a number of levels along the longitudinal axis of the heart, for example between the base and the intermediate fibres, or other devices including passive devices such as small permanent magnets which are suitably positioned and fixed on the heart walls and which therefore move with the heart walls and whose positions and movements can be detected by an external magnetic field sensors which can supply the information relating to both rotational and translational movements of the heart.

## Claims

1. Implantable telemetric device for measuring electromechanical parameters of the heart, comprising a sensor (5) and corresponding processing means for detecting data relating to both the rotation of the heart and the mechanical vibrations which correspond to the first heart sound (FHS) and the second heart sound (SHS), further comprising means which are adapted to use these data for diagnostic and/or therapeutic and/or monitoring purposes, **characterized in that** said sensor (5) is composed of a single miniature gyroscopic sensor of the piezoelectric fork type or a rotation sensor of another type adapted to generate a broadband electrical signal proportional to the rotation rate (RotR) of the heart and proportional to said mechanical vibrations corresponding to the heart sounds, and **in that** the device comprises means which are configured to process the broadband signal generated by said sensor (5), by means of an operation of integration to supply a signal of the angle of rotation of the heart (∫RotRdt) and by means of an operation of derivation to obtain the signal [d(RotR)/dt] of the mechanical vibrations corresponding to the heart sounds (FHS, SHS).

2. Device according to Claim 1, **characterized in that** it comprises means for detecting, on the basis of the end (A) of the first heart sound (End of FHS), the zero reference level of the signal (RotR) produced by said sensor (5) and of the integration corresponding to the signal (∫RotRdt) of the rotation of the heart, in order to enable the signal produced by said sensor (5) to be processed correctly.

3. Device according to Claim 2, **characterized in that** it comprises means for detecting data derived from combination of said signals ∫RotRdt and d(RotR)/dt, such as data relating to any time interval (DI) that may be present between the peak (P2) of said heart rotation signal (∫RotRdt) and the second heart sound (SHS) obtained from the mechanical vibration signal (d(RotR)/dt), and data derived from basic signal processing operations applied to all said signals and **in that** it comprises means for using said data for diagnostic and/or therapeutic and/or monitoring purposes.

4. Device according to Claim 1, **characterized in that** said sensor (5) can be associated with suitable means for acquiring the cardiac ECG of the patient detected by means of suitable electrodes (E1, E2) forming part of the implantable device.

5. Device according to Claim 1, **characterized in that** said sensor (5) can be associated with suitable means for detecting the patient's posture, such as a three-dimensional accelerometer (9) which is sensitive to gravity, so as to associate the detected data with the corresponding and contingent posture of the patient.

6. Device according to Claim 1, in which said sensor (5) can be composed of or associated with one or more suitable motion sensors, such as accelerometer, capable of additionally detecting the translational movements of the heart in the radial and longitudinal directions, or other sensors of the gyroscopic type capable of detecting the rotation of the heart wall at a number of levels along the longitudinal axis of the heart, or other devices, including magnetic devices, whose movement can be detected by an external sensor which can supply the information relating to both the rotational and the translational movements of the heart.

7. Device according to Claim 1, **characterized in that** it comprises at least one said sensor (5) for detecting the rotation and the mechanical vibrations of the heart corresponding to the heart sounds, with a corresponding amplifier (110), said amplifier being connected to a multiplexer (11), to an analogue/digital converter (12), to a RAM (13) and to a control unit (14) which is configured to acquire and processe the data and to operate in a feedback circuit with the upstream units (11, 12 and 13), provision being made to enable said memory unit (13) and control unit (14) to interact with the outside by means of a telemetric unit (15), using an antenna (2).

8. Device according to Claim 7, **characterized in that** said multiplexer (11) is also connected to means, comprised in the device, for acquiring the patient's cardiac ECG, with a corresponding signal amplifier (10) and a three-dimensional accelerometer (9) with a corresponding amplifier (210) which is configured to supply the spatial coordinates relating to the patient's posture during the processing of the data.

9. Device according to Claim 1, **characterized in that** it comprises a unit (18) which is configured to detect the R wave and T wave from the electrocardiographic signal (ECG), the outputs (118, 218) of this unit being enabled to transfer to a subsequent processing unit (19) the marker for the R wave and the marker for the T wave of the ECG respectively, provision being made to connect the broadband heart rotation rate signal (RotR) to the units (20, 21) which, respectively are configured to, supply the derived signal [d(RotR)/dt)] relating to the mechanical vibrations of the heart and the integrated signal (∫RotRdt) relating to the angle of rotation of the heart, the first of these units (20) being connected to said processing unit (19) whose outputs (119, 219) are configured to supply, respectively, the signal relating to the end of the first heart sound (End of FHS) and the signal relating to the start of the second heart sound (Onset of SHS), which, together with the signal received from said second unit (21) and the signal relating to the heart rotation rate (RotR), are adapted to be sent to a final unit (22) which is configured process the various data for the measurement of the parameters relating to the heart rotation, and which has outputs (122, 222, 322, 422, 522, 622) for supplying the measurement of rotation (RE) relating to the ejection phase of the heart chamber, the measurement of the rotation (RF) relating to the refilling phase of the heart chamber, the measurement (RRE) relating to the peak of the rotation rate during ejection, the measurement (RRR) relating to the peak of the rotation rate during the relaxation/refilling of the heart chamber, the measurement of any dyssynchrony interval (DI) that may be present, of any other meaningful parameter derived from basic signal processing operations and the parameters (Pos.) relating to the patient's posture.

10. Device according to Claim 1, **characterized in that** it comprises a casing (1) which is adapted to be placed under the patient's skin (C) and which is covered with silicone or other suitable material, this casing being surrounded by the coil (2) of a system for receiving and transmitting power and data, and housing within it the unit (3) containing the integrated circuits with the necessary hardware and software for data acquisition and transmission and also housing a posture sensor (9) and one of the electrodes (E2) required for the detection of the electrocardiographic signal (ECG) from the patient's heart, said casing (1) being connected to a catheter (4) which is implanted in the patient's body and whose distal end (104) is configured to be placed as closely as possible to the apex of the patient's heart, on the left ventricle side, for example by insertion through the great cardiac vein (VM), said distal end housing said sensor (5) for detecting the electric signal relating to the rotation and to the mechanical vibrations of the heart during the systolic and diastolic phases, at least one other electrode (E1) being positioned in an intermediate part of the catheter (4) and being used in combination with the preceding electrode (E2) for detecting the electrocardiographic signal (ECG).

11. Device according to Claim 10, **characterized in that** it is adapted to interact with an external programming and interrogation or dialogue system, comprised in the device and comprising a reception and transmission antenna (102) connected to a portable data collection unit (6), which can be provided with an alarm signaling device for responding to the detection of any anomalous data, and which, by means of any suitable data transfer means (7), can be connected by a wire or wireless link to an external server (8) with means for data acquisition, for the processing and analysis of the data, and for the generation of commands and feedback signals to the patient.

## Patentansprüche

1. Implantierbare telemetrische Vorrichtung zur Messung elektromechanischer Parameter des Herzens, welche einen Sensor (5) umfasst, sowie entsprechende Verarbeitungselemente zur Ermittlung von Daten, und zwar sowohl die, die die Rotation des Herzens wie die, welche die mechanischen Vibrationen betreffen, die jeweils dem ersten Herzton (FHS) und dem zweiten Herzton (SHS) entsprechen, und die des weiteren Elemente umfasst, die geeignet sind, diese Daten zu diagnostischen und/oder therapeutischen und/oder Überwachungszwecken zu verwenden, **dadurch gekennzeichnet, dass** der genannte Sensor (5) aus einem einzelnen Miniatur-Kreiselsensor des Typs piezoelektrische Gabel oder einem Drehsensor eines anderen Typs besteht, welcher geeignet ist, ein elektrisches Breitbandsignal zu erzeugen, das sich proportional zur Rotationsrate (RotR) des Herzens und proportional zu den genannten mechanischen Vibrationen verhält, die den Herztönen entsprechen, sowie dadurch, dass die Vorrichtung Elemente umfasst, welche entsprechend konfiguriert sind, um das Breitbandsignal zu verarbeiten, das durch den genannten Sensor (5) erzeugt wird, und zwar durch einen Integrationsprozess zur Lieferung eines Signals des Rotationswinkels des Herzens (∫RotRdt) und durch einen Derivationsprozess zur Gewinnung eines Signals [d (RotR)/dt] der mechanischen Vibrationen, die den Herztönen entsprechen (FHS, SHS).

2. Eine Vorrichtung gemäß Anspruch 1, **dadurch gekennzeichnet, dass** sie Elemente umfasst, um auf der Basis des Endes (A) des ersten Herztons (Ende von FHS) den Null-Bezugspegel des Signals (RotR) festzustellen, das von diesem Sensor (5) erzeugt wird, sowie der Integration, die dem Signal (∫RotRdt) der Rotation des Herzens entspricht, um das durch diesen Sensor (5) erzeugte Signal in die Lage zu versetzen, korrekt verarbeitet zu werden.

3. Eine Vorrichtung gemäß Anspruch 2, **dadurch gekennzeichnet, dass** sie Elemente zur Feststellung von Daten umfasst, die aus der Kombination der genannten Signale ∫RotRdt und d(RotR)/dt abgeleitet werden, wie zum Beispiel Daten, die mit einem beliebigen Zeitintervall (DI) zusammenhängen, das zwischen der Spitze (P2) des genannten Herzrotationssignals (∫RotRdt) und dem zweiten Herzton (SHS) liegen kann, die jeweils durch das mechanische Vibrationssignal (d(RotR)/dt) gewonnen werden, sowie Daten, welche von Grundsignalverarbeitungen abgeleitet werden, die auf alle genannten Signale angewendet werden, sowie dadurch, dass sie Elemente zur Verwendung der genannten Daten für diagnostische und/oder therapeutische und/oder Überwachungszwecke umfasst.

4. Eine Vorrichtung gemäß Anspruch 1, **dadurch gekennzeichnet, dass** der genannte Sensor (5) jeweils mit entsprechenden Elementen zur Gewinnung des EKGs des Patienten verbunden werden kann, das mit entsprechenden Elektroden (E1, E2) ermittelt werden kann, die zu der implantierbaren Vorrichtung gehören.

5. Eine Vorrichtung gemäß Anspruch 1, **dadurch gekennzeichnet, dass** der genannte Sensor (5) jeweils mit entsprechenden Elementen zur Feststellung der Stellung des Patienten verbunden werden kann, wie zum Beispiel mit einem dreidimensionaler Beschleunigungsmesser (9), der reagibel auf Schwerkraft ist, so dass die ermittelten Daten mit der entsprechenden und davon abhängenden Stellung des Patienten in Verbindung gebracht werden können.

6. Eine Vorrchtung gemäß Anspruch 1, wobei der genannte Sensor (5) jeweils aus einem beziehungsweise mehreren entsprechenden Bewegungssensoren bestehen oder mit diesen verbunden werden kann, wie zum Beispiel Beschleunigungsmesser, welche zusätzlich die Translationsbewegungen des Herzens in radialen und Längsrichtungen ermitteln können, oder andere Sensoren des Kreiseltyps, die die Rotation der Herzwand auf mehreren Stufen entlang der Längsachse des Herzens ermitteln können, oder andere Vorrichtungen, einschließlich magnetischer Vorrichtungen, deren Bewegung durch einen externen Sensor festgestellt werden kann, welcher die entsprechende Information bezüglich beider, der Rotations- und der Translationsbewegung, liefern kann.

7. Eine Vorrichtung gemäß Anspruch 1, **dadurch gekennzeichnet, dass** sie mindesten einen genannten Sensor (5) zur Feststellung der Rotation und der mechanischen Vibrationen des Herzens umfasst, die jeweils den Herztönen entsprechen, mit einem entsprechenden Verstärker (110), wobei der genannte Verstärker mit einem Multiplexer (11), einem Analog-/Digitalkonverter (12), einem RAM (13) und einer Steuereinheit (14) verbunden ist, welche konfiguriert IST, um die Daten zu erfassen und zu verarbeiten und in einem Rückkopplungskreis mit den stromaufwärts gelegenen Einheiten (11, 12 und 13) zu arbeiten, wobei entsprechende Vorkehrungen getroffen werden müssen, um die genannte Speichereinheit (13) und die Steuereinheit (14) jeweils in die Lage zu versetzen, mit der Außenwelt zu interagieren, und zwar mit Hilfe einer telemetrischen Einheit (15) und bei Verwendung einer entsprechenden Antenne (2).

8. Eine Vorrichtung gemäß Anspruch 7, **dadurch gekennzeichnet, dass** der genannte Multiplexer ebenfalls mit in der Vorrichtung enthaltenen Elementen zur Gewinnung des EKGs des Patienten verbunden ist, mit einem entsprechenden Signalverstärker (10) und einem dreidimensionalen Beschleunigungsmesser (9) mit einem entsprechenden Verstärker (210), welcher konfiguriert ist, um die räumlichen Koordinaten in Bezug auf die Stellung des Patienten während der Verarbeitung der Daten zu liefern.

9. Eine Vorrchtung gemäß Anspruch 1, **dadurch gekennzeichnet, dass** sie eine Einheit (18) umfasst, die konfiguriert ist, um die R-Welle und die T-Welle vom elektrokardiographischen Signal (EKG) zu ermitteln, wobei die Ausgänge (118, 218) dieser Einheit in die Lage versetzt werden, an eine nachgeschaltete Verarbeitungseinheit (19) jeweils den Anzeiger für die R-Welle und den Anzeiger für die T-Welle des EKGs zu übertragen, und entsprechende Vorkehrungen getroffen werden, um das Signal der Breitband-Herzrotationsrate (RotR) mit den Einheiten (20, 21) zu verbinden, welche jeweils konfiguriert sind, um das gewonnene Signal [d(RotRdt)] in Bezug auf die mechanischen Vibrationen und das integrierte Signal (∫Rot/Rtd) in Bezug auf den Rotationswinkel des Herzens zu liefern, wobei die erste dieser Einheiten (20) mit der genannten Verarbeitungseinheit (19) verbunden ist, deren Ausgänge (119, 219) entsprechend konfiguriert sind, um jeweils das Signal in Bezug auf das Ende des ersten Herztons (Ende von FHS) und das Signal in Bezug auf den Beginn des zweiten Herztons (Anfang von SHS) zu liefern, welche, zusammen mit dem Signal, das von der genannten zweiten Einheit (21) empfangen wird, und dem Signal in Bezug auf die Herzrotationsrate (RotR), geeignet sind, zu einer Endeinheit (22) gesendet zu werden, die entsprechend konfiguriert ist, um die mehreren Daten zur Messung der Parameter in Bezug auf die Herzrotation zu verarbeiten, und die entsprechende Ausgänge (122, 222, 322, 422, 522, 622) zur Lieferung der Rotationsmessung (RE) in Bezug auf die Ausstoßphase der Herzkammer, jeweils der Rotationsmessung (RF) in Bezug auf die Füllphase der Herzkammer, der Messung (RRE) in Bezug auf die Spitze der Rotationsrate während des Ausstoßes, der Messung (RRR) in Bezug auf die Spitze der Rotationsrate während der Erschlaffung/Füllung der Herzkammer, der Messung eines beliebigen Dyssynchronie-Intervalls (DI), das vorliegen kann, jedes anderen bedeutungsvollen Parameters, der von Grundsignalverarbeitungen stammt, sowie die Parameter (Pos.) in Bezug auf die Stellung des Patienten aufweist.

10. Eine Vorrchtung gemäß Anspruch 1, **dadurch gekennzeichnet, dass** sie ein Gehäuse (1) umfasst, welches geeignet ist, unter der Haut des Patienten (C) platziert zu werden, und das mit Silikon beziehungsweise einem anderen passenden Material verkleidet ist, wobei dieses Gehäuse von der Spule (2) eines Systems für Empfang und Sendung von Leistung und Daten umgeben ist, und in dem die Einheit (3) untergebracht ist, welche die integrierten Schaltkreise mit der notwendigen Hardware und Software für die Datenerfassung und -Übertragung enthält, und in dem ebenfalls ein Stellungssensor (9) und eine der Elektroden (E2) untergebracht sind, die erforderlich sind für die Feststellung des elektrokardiographischen Signals (EKG) des Herzens des Patienten, wobei das genannte Gehäuse (1) mit einem Katheter (4) verbunden ist, der im Körper des Patienten implantiert ist und dessen distales Ende (104) konfiguriert ist, um so nahe wie möglich an der Spitze des Herzens des Patienten angeordnet zu werden, und zwar an der linken Ventrikelseite, zum Beispiel durch Insertion durch die große Herzvene (VM), wobei das genannte distale Ende den genannten Sensor (5) zur Ermittlung des elektrischen Signals in Bezug auf die Rotation und die mechanischen Vibrationen des Herzens während der systolischen und diastolischen Phasen enthält, und mindestens eine andere Elektrode (E1) in einem Zwischenbereich des Katheters (4) angeordnet ist und zusammen mit der vorausgegangenen Elektrode (E2) zur Ermittlung des elektrokardiographischen Signals (EKG) verwendet wird.

11. Eine Vorrichtung gemäß Anspruch 10, **dadurch gekennzeichnet, dass** sie geeignet ist, mit einem externen Programmier- und Abfrage- beziehungsweise Dialogsystem zu interagieren, das in der Vorrichtung enthalten ist und eine Sende- und Empfangsantenne (102) umfasst, welche mit einer tragbaren Datensammlungseinheit (6) verbunden ist, die mit einer Alarmsignalvorrichtung ausgerüstet werden kann, welche bei Feststellung anomaler Daten anspricht, und die mit Hilfe beliebiger geeigneter Datenübertragungselemente (7) per Kabel oder drahtlos mit einem externen Server (8) mit Elementen zur Datenerfassung verbunden werden kann, zur Verarbeitung und Analyse der Daten und zur Erzeugung von entsprechenden Befehlen und Rückmeldungen an den Patienten.

## Revendications

1. Dispositif télémétrique implantable pour mesurer des paramètres électromécaniques du coeur, comprenant un capteur (5) et des moyens de traitement correspondants pour détecter des données relatives à la fois à la rotation du coeur et aux vibrations mécaniques qui correspondent au premier son cardiaque (FHS) et au deuxième son cardiaque (SHS), comprenant également des dispositifs qui sont adaptés pour utiliser ces données à des fins diagnostiques et/ou thérapeutiques et/ou de surveillance, **caractérisé en ce que** ledit capteur (5) est composé d'un seul capteur gyroscopique miniature de type fourche piézoélectrique ou un capteur à rotation d'un autre type adéquat pour générer un signal électrique à large bande proportionnel à la vitesse de rotation (RotR) du coeur et proportionnel auxdites vibrations mécaniques correspondant aux sons cardiaques et **en ce que** le dispositif comprend des moyens qui sont configurés pour traiter le signal à large bande généré par ledit capteur (5), par l'intermédiaire d'une opération d'intégration pour fournir un signal de l'angle de rotation du coeur (∫RotRdt) et par le biais d'une opération de dérivation pour obtenir le signal [d(RotR)/dt] des vibrations mécaniques correspondant aux sons cardiaques (FHS, SHS).

2. Dispositif selon la revendication 1, **caractérisé en ce qu'**il comprend des moyens pour détecter, sur la base de l'extrémité de fin (A) du premier son cardiaque (Fin de FHS), le niveau de référence zéro du signal (RotR) produit par ledit capteur (5) et de l'intégration correspondant au signal (∫RotRdt) de la rotation du coeur, afin de permettre au signal produit par ledit capteur (5) d'être correctement traité.

3. Dispositif selon la revendication 2, **caractérisé en ce qu'**il comprend des moyens pour détecter des données dérivant de la combinaison desdits signaux ∫RotRdt et d(RotR)/dt, telles que des données relatives à tout intervalle de temps (DI) pouvant se présenter entre la crête (P2) dudit signal de rotation du coeur (∫RotRdt) et le deuxième son cardiaque (SHS) obtenu à partir du signal de vibration mécanique (d(RotR)/dt), et des données résultant d'opérations de base de traitement des signaux appliquées à chacun desdits signaux et **en ce qu'**il comprend des moyens pour utiliser lesdites données à des fins diagnostiques et/ou thérapeutiques et/ou de surveillance.

4. Dispositif selon la revendication 1, **caractérisé en ce que** ledit capteur (5) peut être associé à des dispositifs appropriés pour l'acquisition de l'ECG cardiaque du patient détecté au moyen d'électrodes appropriées (E1, E2) faisant partie du dispositif implantable.

5. Dispositif selon la revendication 1, **caractérisé en ce que** ledit capteur (5) peut être associé à des moyens adaptés pour détecter la posture du patient, tels qu'un accéléromètre à trois dimensions (9) qui est sensible à la gravité, de manière à associer les données détectées à la posture contingente et correspondante du patient.

6. Dispositif selon la revendication 1, **caractérisé en ce que** ledit capteur (5) peut être constitué de ou associé à un seul ou à plusieurs capteurs de mouvement adéquats, tels que des accéléromètres, en mesure de détecter également les mouvements de translation du coeur dans les directions radiale et longitudinale, ou d'autres capteurs de type gyroscopique en mesure de détecter la rotation de la paroi cardiaque à un certain nombre de niveaux le long de l'axe longitudinal du coeur, ou d'autres dispositifs, y compris des dispositifs magnétiques, dont le mouvement peut être détecté par un capteur externe qui peut fournir les informations relatives à la fois aux mouvements de translation et de rotation du coeur.

7. Dispositif selon la revendication 1, **caractérisé en ce qu'**il comprend au minimum un dit capteur (5) pour détecter la rotation et les vibrations mécaniques du coeur correspondant aux sons cardiaques, avec un amplificateur correspondant (110), ledit amplificateur étant relié à un multiplexeur (11), à un convertisseur analogique/numérique (12), à une mémoire RAM (13) et à une unité de commande (14) qui est configurée pour acquérir et traiter les données et fonctionner dans un circuit de rétroaction avec les unités placées en amont (11, 12 et 13), l'alimentation étant réalisée afin de permettre auxdites unité de mémoire (13) et unité de commande (14) d'interagir avec l'extérieur au moyen d'une unité télémétrique (15), en utilisant une antenne (2).

8. Dispositif selon la revendication 7, **caractérisé en ce que** ledit multiplexeur (11) est également relié à des moyens, compris dans le dispositif, d'acquisition de l'ECG cardiaque du patient, avec un amplificateur de signal correspondant (10) et un accéléromètre à trois dimensions (9) avec un amplificateur correspondant (210) qui est configuré pour fournir les coordonnées spatiales relatives à la posture du patient pendant le traitement des données.

9. Dispositif selon la revendication 1, **caractérisé en ce qu'**il comprend une unité (18) qui est configurée pour détecter l'onde R et l'onde T à partir du signal électrocardiogramme (ECG), les sorties (118, 218) de cette unité étant activées pour transférer à une unité de traitement ultérieure (19), respectivement le marqueur de l'onde R et le marqueur de l'onde T de l'ECG, l'alimentation étant réalisée pour connecter le signal à large bande de la vitesse de rotation du coeur (RotR) aux unités (20, 21) qui, respectivement, sont configurées pour fournir le signal dérivé [d(RotR)/dt)] se rapportant aux vibrations mécaniques du coeur et le signal intégré (∫RotRdt) se rapportant à l'angle de rotation du coeur, la première de ces unités (20) étant connectée à ladite unité de traitement (19) dont les sorties (119, 219) sont configurées pour fournir, respectivement, le signal se rapportant à la fin du premier son cardiaque (Fin de FHS) et le signal se rapportant au début du deuxième son cardiaque (Début de SHS), qui, avec le signal reçu de ladite deuxième unité (21) et le signal se rapportant à la vitesse de rotation du coeur (RotR), sont adéquats pour être envoyés à une unité finale (22) qui est configurée pour traiter les différentes données de mesure des paramètres se rapportant à la rotation du coeur, et qui présente des sorties (122, 222, 322, 422, 522, 622) pour fournir la mesure de la rotation (RE), se rapportant à la phase d'éjection de la cavité cardiaque, la mesure de la rotation (RF) se rapportant à la phase de remplissage de la cavité cardiaque, la mesure (RRE), relative à la pointe de la vitesse de rotation lors de l'éjection, la mesure (RRR) relative à la pointe de la vitesse de rotation au cours de la détente/du remplissage de la cavité cardiaque, la mesure de tout intervalle d'asynchronisme (DI) qui peut être présent, de tout autre paramètre significatif provenant des opérations de base de traitement du signal et les paramètres (Pos.) se rapportant à la posture du patient.

10. Dispositif selon la revendication 1, **caractérisé en ce qu'**il comprend un boîtier (1) qui est adéquat pour être placé sous la peau du patient (C) et qui est recouvert de silicone ou d'un autre matériau approprié, ce boîtier étant entouré par la bobine (2) d'un système pour recevoir et transmettre de l'énergie et des données, et accueillant en son intérieur l'unité (3) contenant des circuits intégrés avec le matériel informatique et le logiciel nécessaires pour l'acquisition et la transmission de données et accueillant également un capteur de position (9) et l'une des électrodes (E2) nécessaires pour la détection du signal électrocardiogramme (ECG) provenant du coeur du patient, ledit boîtier (1) étant relié à un cathéter (4) qui est implanté dans le corps du patient et dont l'extrémité de fin distale (104) est configurée pour être placée aussi près que possible du sommet du coeur du patient, sur le côté du ventricule gauche, par exemple par l'introduction à travers la grande veine cardiaque (VM), ladite extrémité distale accueillant ledit capteur (5) pour détecter le signal électrique se rapportant à la rotation et aux vibrations mécaniques du coeur pendant les phases systoliques et diastoliques, au minimum une autre électrode (E1) étant positionnée dans une partie intermédiaire du cathéter (4) et étant utilisée en combinaison avec l'électrode précédente (E2) pour détecter le signal électrocardiogramme (ECG).

11. Dispositif selon la revendication 10, **caractérisé en ce qu'**il est adapté pour interagir avec un système de dialogue ou d'interrogation et de programmation externe, compris dans le dispositif et comprenant une antenne de réception et de transmission (102) reliée à une unité portable de recueil des données (6), pouvant être pourvue d'un dispositif d'alarme pour répondre à la détection de toutes données anormales, et qui, par l'intermédiaire de tout dispositif de transfert approprié de données (7), peut être connecté par une connexion avec fil ou sans fil à un serveur externe (8) avec des dispositifs d'acquisition des données, pour le traitement et l'analyse des données, et pour générer des commandes et des signaux de rétroaction au patient.
